(19) Europäisches Patentamt
European Patent Office
Office européen des brevets

(11) EP 0 525 836 B1

(12) # EUROPEAN PATENT SPECIFICATION

(45) Date of publication and mention of the grant of the patent:
**09.12.1998 Bulletin 1998/50**

(21) Application number: **92118167.3**

(22) Date of filing: **10.09.1987**

(51) Int Cl.[6]: **C12Q 1/00**, C12N 1/20, C12Q 1/48, C12Q 1/34, C12Q 1/32 // (C12N1/20, C12R1:07)

(54) **Assay method using novel NAD synthetase**

Neue NAD-Synthetase gebrauchende Methode

Méthode d'essai utilisant une nouvelle NAD synthétase

(84) Designated Contracting States:
**DE FR IT**

(30) Priority: **10.09.1986 JP 213679/86**
**30.07.1987 JP 191018/87**

(43) Date of publication of application:
**03.02.1993 Bulletin 1993/05**

(62) Document number(s) of the earlier application(s) in accordance with Art. 76 EPC:
**87308005.5 / 0 260 137**

(73) Proprietor: **ASAHI KASEI KOGYO KABUSHIKI KAISHA**
**Osaka (JP)**

(72) Inventors:
- **Takahashi, Mamoru**
  **Sunto-gun, Shizuoka-ken (JP)**
- **Misaki, Hideo**
  **Tagata-gun, Shizuoka-ken (JP)**
- **Imamura, Shigeyuki**
  **Tagata-gun, Shizuoka-ken (JP)**
- **Matsuura, Kazuo**
  **Tagata-gun, Shizuoka-ken (JP)**

(74) Representative: **Woods, Geoffrey Corlett et al**
**J.A. KEMP & CO.**
**14 South Square**
**Gray's Inn**
**London WC1R 5LX (GB)**

(56) References cited:
**FR-A- 2 545 504**

- **EXPERIENTIA vol. 29, no. 8, 15 August 1973, pages 941 - 942 H. HENGARTNER ET AL. 'ISOLATION OF DIFFERENT THERMOPHILIC ENZYMES FROM BACILLUS STEAROTHERMOPHILUS'**
- **THE JOURNAL OF BIOLOGICAL CHEMISTRY vol. 247, no. 15, 10 August 1972, pages 4794 - 4802 C. K. YU ET AL. 'PURIFICATION AND PROPERTIES OF YEAST NICOTINAMIDE ADENINE DINUCLEOTIDE SYNTHETASE'**
- **THE JOURNAL OF BIOLOGICAL CHEMISTRY vol. 242, no. 3, 10 February 1967, pages 385 - 392 R. L. SPENCER ET AL. 'BIOSYNTHESIS OF DIPHOSPHOPYRIDINE NUCLEOTIDE'**
- **THE JOURNAL OF BIOLOGICAL CHEMISTRY vol. 236, no. 5, May 1961, pages 1494 - 1497 J. IMSANDE 'PATHWAY OF DIPHOSPHOPYRIDINE NUCLEOTIDE BIOSYNTHESIS IN ESCHERICHIA COLI.'**

EP 0 525 836 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

This invention relates to a method of assaying a specimen for ATP, deamide-NAD, or ammonia using a NAD synthetase.

More particularly, the present invention relates a method using a NAD synthetase ( hereinafter sometimes referred to as the present NAD synthetase ) which at least catalyses the reaction ( a ) as defined hereinbelow in the presence of $Mg^{++}$ or $Mn^{++}$ ions and which does not catalyse the reaction ( b ) as defined hereinbelow in the presence of $Mg^{++}$ ions, utilizes ammonia including ammonium ions, and does not utilize glutamine and asparagine which method comprises incubating the specimen with the present NAD synthetase.

$$\text{( a ) ATP + deamide-NAD} + NH_3 \xrightleftharpoons{Mg^{++} \text{ or } Mn^{++}} \text{AMP + PPi + NAD}$$

$$\text{( b ) ATP + deamide-NAD + L-Gln} + H_2O \xrightleftharpoons{Mg^{++}} \text{AMP + PPi + NAD + L-Glu}$$

NAD synthetases are known to exist in rat liver [ J. Biol. Chem. 233 , 493 - 500 ( 1958 )] , porcine liver [ ibid., 236 , 525 - 530 ( 1961 ) ], yeast [ ibid., 247 , 4794 - 4802 (1972)] and E. Coli [ ibid., 236 , 1494 - 1497 (1961) and 242, 385 - 392 (1967)] .

Said NAD synthetases are classified as NAD synthetase ( EC 6.3.1.5 ) which catalyses the reaction :

$$\text{ATP + deamide-NAD} \div NH_3 \xrightleftharpoons{Mg^{++}} \text{AMP + PPi + NAD}$$

and NAD synthetase ( EC 6.3.5.1 ) which catalyses the reaction :

$$\text{ATP + deamide-NAD + L-Gln} \div H_2O \xrightleftharpoons{Mg^{++}} \text{AMP + PPi + NAD + L-Glu}$$

These NAD synthetases utilize $NH_3$ or an amide of L-Gln, and are differentiated by the inhibitory action of azaserine.

The km-value of NAD synthetase ( EC 6.3.1.5 ) is reported as $6.5 \times 10^{-5}$ M ( $NH_4^+$ ) and $1.6 \times 10^{-2}$ M ( L-Gln ) [ J. Biol., Chem., 1494 - 1497, ( 1961 ), ibid., 242, 385 - 392 ( 1967 ) ] and the Km-value of NAD synthetase ( EC 6.3.5.1 ) is $6.4 \times 10^{-8}$ M ( $NH_4^+$ ) and $5 \times 10^{-8}$ M ( L-Gln ) [ J. Biol. Chem. 247, 4794 - 4802 ( 1972 ), ibid. 233, 493 - 500 ( 1958 ) ] .

An assay method for NAD synthtase has been reported, in which the NAD generated is reduced by alcohol dehydrogenase ( EC 1.1.1 ) and the absorbancy of the generated and reduced NAD ( hereinafter referred to as NADH ) is spectorophotometrically measured at 340 nm or the amount of generated NAD is measured by fluorometry.

We have reported as assay method for a component in a specimen, said component being ATP, deamide-NAD or an amide donor such as $NH_3$, L-glutamine or L-asparagine, which comprises, as a main reaction step, incubating the specimen with the known NAD synthetase ( EC 6.3.1.5 or EC 6.3.5.1 ) in the presence of ATP, deamide-NAD, an amide donor and $Mg^{++}$ to generate NAD, performing a coenzyme cycling reaction by combining the oxidation-reduction reaction system with coenzyme NAD and the oxidation-reduction reaction system with coenzyme reduced NAD, and

measuring the amount of a component consumed or generated in the cycling reaction (JP-A-59-198995 ).

As explained, the known NAD synthetase utilizes the amide donor substrate L-glutamine. A NAD synthetase which at least catalyses the reaction ( a ) as defined above in the presence of $Mg^{++}$ or $Mn^{++}$ ions and does not catalyse the reaction ( b ) as defined above in the presence of $Mg^{++}$ ions, utilizes ammonia including ammonium ions and does not utilize glutamine and asparagine, is not known.

Furthermore the known NAD synthetase has substrate specificity for L-glutamine, and so $NH_3$ cannot be measured in the presence of L-glutamine.

We have found that Bacillus sp. H-804, isolated from a soil sample obtained from hot spring water at Tanoyu-machi, Beppu-shi, Oita-ken, Japan, produces the present NAD synthetase.

The present invention provides a method of assaying a specimen for ATP, deamide-NAD, ammonia including ammonia in the form of an ammonium ion or a component of a reaction system in which ATP, deamide-NAD or ammonia including ammonia in the form of an ammonium ion is generated or consumed, which method comprises:

(i) incubating the specimen with a NAD synthetase having the following properties:

(1) Action:
at least catalyses in the presence of $Mg^{++}$ or $Mn^{++}$ ion the reaction (a):

$$\text{(a)}\quad ATP + \text{deamide-NAD} + NH_3 \xrightleftharpoons{Mg^{++}\text{ or }Mn^{++}} AMP + PPi + NAD$$

and does not catalyse in the presence of $Mg^{++}$ ion the reaction (b):

$$\text{(b)}\quad ATP + \text{deamide-NAD} + \text{L-Gln} + H_2O \xrightleftharpoons{Mg^{++}} AMP + PPi + NAD + \text{L-Glu}$$

(2) Substrate specificity:

utilises ammonia, including ammonia in the form of an ammonium ion, as a substrate but does not utilise glutamine or asparagine as a substrate;

in the presence of $Mg^{++}$ or $Mn^{++}$ ions and the two of ATP, deamide-NAD and ammonia which may be in the form of an ammonium ion which are not being assayed for or which are not generated or consumed by the said reaction system, to generate thereby NAD;

(ii) subjecting the thus-generated NAD to a coenzyme cycling reaction comprising reducing said generated NAD to reduced NAD and oxidizing the thus-generated reduced NAD to NAD; and

(iii) measuring a compound which is consumed or generated in said cycling reaction.

Taxonomical properties of the strain which produces the present NAD synthetase are as follows.

A. Morphological properties :

Observed by microscope on nutrient agar slant medium at 50°C for 1 - 3 days cultivation.

| | |
|---|---|
| 1. Form and arrangement | Round edges, straight or slightly curved bacilli, single or binary chains. |
| 2. Size | 0.5 to 1.0 × 1.6 to 5.5 μm. |
| 3. Motility | motile by peritrichous flagella. |
| 4. Spores | forms center or subterminal, 0.8 to 1.0 × 1.0 to 1.5 μm, swelling the sporangia. |

B. Growth on various media ( at 50°C ):

| | |
|---|---|
| 1. Nutrient agar plate | Colonies grayish to pale yellowish white and semi-transparent, linear growth. weak growth. No soluble pigment formation. |
| 2. Nutrient agar slant | Grayish to pale yellowish and semi-transparent, round plain colonies. No solube pigment formation. |
| 3. Bouillon agar | Uniformly turbid, good growth. 2 to 3 days forms precipitation. |
| 4. BCP milk | No changes. |

C. Physiological properties ( +: positive, -: negative):

| | |
|---|---|
| Gram's stain | + |
| KOH reaction | - |
| acid-fast staining | - |
| capsulation | - |
| anaerobic growth | - |
| catalase production | + ( weak ) |
| oxidase production | + ( weak ) |
| urease formation | |
| ( SSR medium ) | - |
| ( Chris. medium ) | - |
| lecithinase production | no growth |
| gelatin hydrolysis | + |
| starch hydrolysis | + |
| casein hydrolysis | + |
| esculin hydrolysis | + |
| arginine hydrolysis | - |
| cellulose hydrolysis | - |
| indole production | - |
| $H_2O$ production | + ( lead acetate paper ) |
| acetonin production | - |
| MR test | - |
| nitrate reduction | - |
| growth on no NaCℓ added medium | + |
| growth on 0.1% NaC ℓ added medium | + |
| growth on 0.25% NaC ℓ added medium | + |
| growth at 65 °C | + |
| growth at 50 °C | + |
| growth at 37 °C | - |
| growth at pH 9.0 | - |
| growth at pH 8.0 | + |
| growth at pH 5.6 | + |
| growth at pH 4.8 | - |

| acid formation from sugar* ( no gas formation): | | | |
|---|---|---|---|
| adonitol | - | D-mannose | + |
| L( +)arabinose | + | melezitose | + |
| cellobiose | + | melibiose | + |
| dulcitol | - | raffinose | + |
| meso-erylthritol | - | rhamnose | - |
| D-fructose | - | D-ribose | + |
| fucose | - | salicine | - |
| D-galactose | + | L-sorbose | - |
| D-glucose | + | sorbitol | - |
| glycerin | + | starch | + |
| inositol | - | saccharose | + |
| inilin | - | trehalose | + |
| lactose | + | D-xylose | |
| maltose | - | | |
| D-mannitol | + | | |
| OF test ( Hugh-Leifson medium ) | | NT( no change ) | |
| OF test ( modified )** | | 0 ( oxidation ) | |

* basal medium : ( Sugar added ammonium medium ) (ASS)
$(NH_4)_2HPO_4$ 1.0 g KCℓ 0.2 g
$MgSO_4 \cdot 7H_2O$ 0.2 g Yeast ex 1.0 g
Agarose 3.0 g BTB 0.02 g
Distilled water 1000 ml pH 7.0

** modified medium : added glucose 10.0 g to basal medium.

Utilization test :

| | Simmons medium | Christensen medium |
|---|---|---|
| citrate | - | - |
| malonate | - | - |
| gluconate | - | + |
| propionate | - | - |
| malainate | - | - |
| succinate | - | + |
| malate | + | + |

According to the above taxonomical properties, strain H-804 is a bacterium having the characteristics of round edges, straight or slightly curved bacilli, Gram-positive, sporulating at 0.5 to 1.0 × 1.6 to 5.5 μm thermophillic bacterium, weak catalase and oxidase production, no motile and oxidative degradation of sugar ( glucose ). Comparing these taxonomical properties with " Bergey's Manual ", 8th Ed., 1974, " Manual of Medicinal Bacteriology ", 2nd Ed., 1974 and " Agriculture Handbook ", p. 427, " The genus Bacillus ", the present strain is characterized by spore formation and aerobic growth and so belongs to the genus Bacillus . Among strains belonging to genus Bacillus which are thermophilic and thermotolerant are ( a ) Bacillus subtilis, ( b ) Bacillus coagulance, ( c ) Bacillus liqueniformis , ( d ) Bacillus brevis and ( e ) Bacillus stearothermophilus. The present strain can grown at above 30°C, and therefore is either ( A ) Bacillus stearothermophilus or ( B ) Bacillus brevis. Comparison of these strains is as follows : [ +: positive, -: negative, d: different in strain ]

| | H - 804 | ( A ) | ( B ) |
|---|---|---|---|
| Size ( μm ) | | | |
| width | 0.5 to 1.0 | 0.5 to 0.1 | 0.6 to 0.9 |
| length | 1.6 to 5.5 | 2 to 3.5 | 1.5 to 4.0 |
| Gram's strain | + (decolor) | indefinite | indefinite |

(continued)

| | H - 804 | ( A ) | ( B ) |
|---|---|---|---|
| sporulation | - | + | + |
| swelling of spores | + | + | + |
| motility | - | + | + |
| catalase production | (+) | + | + |
| anaerobic growth | - | - | - |
| acetoin production | - | - | - |
| growth temp. | | | |
| max. ( °C ) | > 65 | 65 to 75 | 40 to 60 |
| min. ( °C) | > 37 | 30 to 45 | 10 to 35 |
| growth at pH 5.7 | + | - | d |
| growth on 5% NaC ℓ added medium | - | d | - |
| acid formation from sugar glucose | + | + | + |
| arabinose | + | d | - |
| xylose | + | d | - |
| mannitol | + | d | d |
| gas production from sugar | - | - | - |
| starch hydrolysis | + | + | d |
| citrate utilization | - | - | d |
| nitrate reduction | - | d | d |
| indole production | - | - | - |
| caseine hydrolysis | + | d | + |

The properties of the present strain are quite similar to those of Bacillus stearothermophilus , and it is reported that a non-motile strain can easily be obtained. A comparison of the other properties suggest an identification of the present strain as Bacillus stearothermophilus. Thus the present strain is designated Bacillus stearothermophilus H-804. The strain has been deposited in the Fermentation Research Institute under the number FERM BP-1408.

In the present invention the above strain is an example of a NAD synthetase producing microorganisms belonging to genus Bacillus, but any strain which belongs to genus Bacillus and which produces the present NAD-synthetase can be used.

An artificial mutant can be prepared by isolating the DNA which bears the genetic code of the present NAD synthetase by recombinant DNA technology from the present NAD synthetase producing microorganisms, cloning the said DNA into another microorganism which does not produce the present NAD synthetase, and expressing the present NAD synthetase producing activity. An assay method using an enzyme produced by such a mutant is included in the scope of the present invention.

The NAD-synthetase-producing microorganism belonging to the genus Bacillus may be cultured in a conventional medium for enzyme production. Cultivation is carried out in liquid or solid culture; submerged aeration culture is preferred for industrial production.

The nutrient sources of the medium may be conventional media for microorganism cultivation. The carbon sources are assimilable carbon compounds, for example glucose, sucrose, lactose, maltose, starch, dextrin, molasses or glycerin. The nitrogen sources are assimilable nitrogen sources such as corn steep liquor, soybean powder, cotton seed powder, wheat gluten, peptone, meat extract, yeast extract or casein hydrolyzate. Salts such as magnesium, potassium, sodium, zinc, iron or manganese salts and phosphates or halides can be used.

The culturing temperature for Bacillus stearothermophilus H-804 can be chosen for its growth and the production of the enzyme. The temperature is, for example, from 48 to 70 °C preferably from 55 to 60 °C. The culturing time can be varied depending on the culturing conditions, and is generally from 10 to 20 hours. The cultivation should naturally be stopped upon the maximum production of the enzyme. The aeration agitation is usually from 200 to 400 r.p.m.

Since the enzyme is an endo-enzyme, the cultured cells are collected by filtration or centrifugation, and the collected cells are mechanically disrupted by ultrasonication. French pressing or glass-beads treatment, or enzymatic digestion by a lysozyme, with the addition, if necessary, of surface active agents such as Triton X-100 ( trade name ) or Adekatol S0-120 (trdae name) may be carried out.

The enzyme solution is, with or without concentration, subjected to salting-out by adding soluble salts such as ammonium sulfate, or treated by adding a water-miscible organic solvent such as methanol, ethanol, acetone or isopropanol to precipitate the enzyme. The precipitate is dissolved in water or a buffer solution, dialyzed if necessary, and chromatographed by an ion exchange resin such as DEAE-Sephadex, DEAE-Sepharose, carboxymethyl cellulose, carboxymethyl Sepharose or carboxymethyl Sephadex, or by gel-filtration using a molecular seive such as Sephadex G-200, Sephadex CL-6B OR Sephacryl S-200 ( trade names ). If required stabilizing agents may be added and lyophilization carried out to prepare a purified enzyme.

The biochemical properties of the above NAD synthetase are as follows :

(1) Molecular weight : approximately 50,000 [gel filtration by polyvinyl-gel ( trade name GPC 3,000 SW: Toyo Soda Co., ), using a column ( 7.5 mm ID × 60 cm ). Standard proteins : aldorase (rabbit muscle, N.W. 150,000 ), bovine serum albumin ( M.W. 67,000 ) , avoalbumin ( egg : M.W. 45,000 ) and cytochrome C ( horse meat, M. W. 13,000 )]

(2) Isoelectric point:approximately pH 5.3 (electrophoresis using carrier-Ampholite, using a colum (24 × 30 cm LKB Co. ), 700V, 48 hrs. cutting each 2 cm, measured pH and activity ) $Mg^{++}$ or + $Mn^{++}$

(3) Activity :

$$ATP+deamide\text{-}NAD + NH_3 \rightarrow ATP+PPi + NAD$$

(4) Substrate specificity : $NH_3$ including ammonium ion.

In any assay method for enzyme activity hereinafter described, $(NH_4)_2SO_4$ 25 mM may be replaced by L-valine, L-homosirine, L-serine, L-alanine, L-methine, L-tyrosine, L-threonine, L-leucine, L-isolucine, L-arginine, L-phenylalanine, L-histidine, L-asparagine or L-glutamine, and the enzymatic activity measured. The relative activity of the enzyme on these amino acids is 0.0 when that on $(NH_4)_2SO_4$ is 100. Therefore the present enzyme can only utilize ammonia including ammonium ions and does not utilize at least the abovementioned amino acids.

(5) Optimum pH :

Reaction medium I, identified hereinafter under the assay method for enzymatic activity, is mixed with dimethylglutarate-NaOH buffer ( pH 5.0 to 7.0 ), Tris-HCℓ buffer (pH 6.5 to 9.0) and glycine-NaOH buffer ( pH 8.5 to 10.0). The enzyme activity is measured after stopping the enzyme action by heating at 100 °C for 10 minutes. The optimum pH is from 8.5 to 10.0.

(6) pH-stability :

The enzyme is dissolved in 50 mM dimethylglutarate-NaOH buffer ( pH 5.0 to 7.0 ), Tris-HCℓ buffer ( pH 6.5 to 9.0), or glycine-NaOH buffer ( pH 8.5 to 10.0 ), and the solution is incubated at 60 °C for 15 minutes. The remaining enzyme activity is measured by an assay. The enzyme is stable at a pH of from 7.5 to 9.0.

(7) Heat stability :

The enzyme dissolved in 50 mM Tris-HCℓ buffer ( pH 8.0 ) is held at various temperature for 15 minutes each and the remaining enzyme activity is measured. The enzyme is stable at least below 60°C.

(8) optimum temperature :

The enzyme mixed with reaction medium I hereinafter defined is incubated at temperatures of 50°C , 55°C , 60°C, 65°C and 70 °C for 10 minutes, immediately cooled thereafter, reaction medium II added thereto at 37 °C, and the enzyme activity measured. The optimum temperature is approximately 60°C.

(9) Effect of enzyme activator and inhibitor :

In the enzyme activity assay, each metallic ion ( 5 mM ), EDTA ( 20 mM ) or each surface active agent ( 0.1 % ) in Table 1 is added separately to the reaction medium I, and the enzyme activity is measured. The results are shown in Table 1. The enzyme is inhibited by Ni-iron ( $NiC\ell_2$, 5 mM ) and no activity is observed in EDTA ( 20 mM ).

Furthermore $MgC\ell_2$ ( 5 mM ) is replaced by $MnC\ell_2$( 3 mM ) in the reagent identified in the enzyme assay method hereinafter defined. The activity of the enzyme is increased to 150 % in the presence of $MnC\ell_2$ ( 3 mM ) as compared

with $MgC\ell_2$ ( 5mM ).

Table 1

| | Concentration | Relative activity |
|---|---|---|
| No addition * | - | 100 ( % ) |
| $LiC\ell$ | 5 mM | 100 |
| $KNO_3$ | " | 99.2 |
| KCN | " | 100 |
| $NaC\ell$ | " | 102.4 |
| $NaNO_3$ | " | 96.8 |
| $NaN_3$ | " | 102.4 |
| $CaC\ell_2$ | " | 48.4 |
| $BaC\ell_2$ | " | 89.5 |
| $MnC\ell_2$ | " | 54.0 |
| $NiC\ell_2$ | " | 7.3 |
| $CsC\ell$ | " | 100 |
| $A\ell C\ell_3$ | " | 73.4 |
| $FeC\ell_3$ | " | 88.4 |
| EDTA | 20 mM | 0 |
| Triton X-100 | 0.1 % | 109.6 |
| Nonidet P-40 | " | 103.9 |
| Adekatol PC-8 | " | 98.7 |
| Adekatol SO-120 | " | 99.1 |
| Deoxycholate | " | 104.7 |
| Brig 35 | " | 102.2 |

* $Mg C\ell_2$ ( 5 mM ) is added. Other reactions are carried out with $Mg^{++}$ and each additive.

(10) Assay method of enzyme activity :

Enzyme activity assay :

Reaction medium I

50 mM Tris-HC $\ell$ buffer pH 8.0
1 mM K C $\ell$
5 mM MgC $\ell_2$
0.05 % bovine serum albumin
2 mM ATP
0.5 mM deamide-NAD
25 mM ( $NH_4$ )$_2$ $SO_4$

Reaction medium II

50 mM Tris-HC$\ell$ buffer pH 8.0
10 U diaphorase ( Toyo Jozo Co. from genus Bacillus)
3 % ethanol
10 U alcohol dehydrogenase/m $\ell$ ( Toyo Jozo, yeast )
0.025 % NTB ( nitrotetrazolium blue )
0.1 % Triton X-100
10 mM EDTA

Reaction medium I ( 0.3 m$\ell$ ) in a test tube is pre-incubated at 37 °C, and the enzyme solution ( 5 μ $\ell$ ) is added thereto. The mixture is then incubated at 37 °C for exactly 10 minutes.

Reaction medium II ( 0.8 m$\ell$ ) is added thereto to stop the reaction and simultaneously to start the cycling reaction at 37°C for exactly 5 minutes. After stopping the cycling reaction by adding 0.1 N-HC $\ell$( 2.0 m$\ell$) , the absorbency at

550 nm is measured to calculate the enzyme activity. The enzyme activity is calculated by the following equation :

$$\text{NAD synthetase activity ( mU/ m}\ell\text{ ) =}$$

$$\frac{\Delta A_{550}}{\Delta S_{550}} \times \frac{1.0}{0.005} \times \frac{f}{10}$$

wherein

ΔA: absorbency of specimen,
ΔS: absorbency of standard solution ( 0.1 mM NAD ),
0.005: specimen volume ( mℓ )
10 : reaction time,
f : dilution ration.

The reaction system used in the present invention is summarized as follows.

$$ATP + \text{deamide-NAD} + NH_3 \xrightleftharpoons{Mg^{++}\ \text{or}\ Mg^{++}} AMP + PPi + NAD$$

An assay specimen for use in the assay of the present invention can be a specimen containing at least ATP, deamide-NAD or ammonia including ammonium ions, for example a specimen previously containing any one of the components or a specimen containing generated or consumed components.

A preferred example of the enzyme reaction system is a reaction system which consumes or generates ATP, deamide-NAD or $NH_3$ including ammonium ions, without the coenzyme NAD and NADH, as in the following examples.

(1) Enzymatic reaction systems which generate ATP:

1) creatine kinase ( EC 2.7.3.2 ):

$$\text{creatine phosphate} + ADP \xrightarrow[Mg^{++}]{\text{reducing agent}} \text{creatine} + ATP$$

reducing agent : β-mercapto ethanol, reduced glutathione, cysteine, N-acetylcysteine, dithiothreitol.

2) pyruvate kinase ( EC 2.7.1.40 ):

$$\text{phosphoenol pyruvate} + ADP \xrightarrow[\text{or}\ Mg^{++},\ K^{+}]{Mg^{++}\ \text{or}\ Mn^{++}} \text{pyruvate} + ATP$$

3) acetate kinase ( EC 2.7.2.1 ) :

$$\text{acethylphosphate} + \text{ADP} \xrightarrow{Mg^{++} \text{ or } Mn^{++}} \text{acetate} + \text{ATP}$$

4) carbamate kinase ( EC 2.7.2.2 ):

$$\text{carbamoylphosphate} + \text{ADP} \xrightarrow{Mg^{++}} NH_3 + CO_2 + \text{ATP}$$

5) aspartate kinase ( EC 2.7.2.4 ):

$$\text{4-phospho-L-aspartate} + \text{ADP} \xrightarrow{Mg^{++}} \text{L-aspartate} + \text{ATP}$$

6) phosphoglycerate kinase ( EC 2.7.2.3 ):

$$\text{1,3-diphospho-D-glycerate} + \text{ADP} \xrightarrow{Mg^{++} \text{ or } Mn^{++}} \text{3-phospho-D-glycerarte} + \text{ATP}$$

7) arginine kinase ( EC 2.7.3.3 ):

$$\text{arginine phosphate} + \text{ADP} \xrightarrow{Mg^{++} \text{ or } Mn^{++}} \text{L-arginine} + \text{ATP}$$

(2) Enzymatic reaction systems which utilize ammonium-generating soluble ammonium salts or $NH_3$ :

1) Examples of water-soluble ammonium salts are inorganic or organic ammonium salts which generate ammonium ions such as ammonium chloride, aqueous ammonia, ammonium sulfate, ammonium nitrate, ammonium acetate and ammonium citrate.

2) nicotine amidase ( EC 3.5.1.19 ):

$$\text{nicotine amide} + H_2O \rightarrow \text{nicotinate} + NH_3 + H^+$$

3) glutamyl-peptide-glutaminase ( EC 3.5.1.44) :

$$\text{L-glutaminyl-peptide} + H_2O \rightarrow \text{L-glutamyl-peptide} + NH_3$$

4) arginine deaminase ( EC 3.5.3.6 ):

$$\text{L-arginine} + H_2O \rightarrow \text{citrulline} + NH_2 + H^+$$

5) guanidine deaminase ( EC 3.5.4.3 ):

$$guanine + H_2O \rightarrow xanthine + NH_3 + H^+$$

6) adenosine deaminase ( EC 3.5.4.4 ):

$$adenosine + H_2O \rightarrow inosine + NH_3 + H^+$$

7) creatinine deaminase ( EC 3.5.4.21 ):

$$creatinine + H_2O \rightarrow N\text{-}methylhydantoin + NH_3 + H^+$$

8) threonine dehydratase ( EC 4.2.1.16 ):

$$L\text{-}threonine + H_2O \rightarrow 2\text{-}oxobutyrate + CO_2 + NH_3 + H^+$$

9) aspartate ammonium-liase ( EC 4.3.1.1 ):

$$L\text{-}aspartate \rightarrow fumarate + NH_3 + H^+$$

10) L-methionine- $\gamma$-liase ( EC 4.4.41.11 ):

$$L\text{-}methionine + H_2O \rightarrow 2\text{-}oxobutyrate + methanethiol + NH_3 + H^+$$

11) methylaminoglutamate methyl transferase ( EC 2.1.1.21):

$$N\text{-}methylglutamate + NH_3 + H^+ \rightleftarrows glutamate + methylamine$$

(3) Enzymatic reaction system utilizing AMP for assaying deamide-NAD or $NH_3$ including ammonium ion in a specimen: Adenylate kinase ( EC 2.7.4.3 ):

$$AMP + ATP \rightleftarrows ADP + ADP$$

pyruvate kinase ( EC 2.7.1.40 )

$$ADP + phosphenol\ pyruvate \rightleftarrows ATP + pyruvate$$

①pyruvate oxidase ( EC 1.2.3.3 ):

$$pyruvate + pi + O_2 + H_2O \rightleftarrows acetyl\ phosphate + CO_2 + H_2O_2$$

The generated $H_2O_2$ is measured in the presence of peroxidase, and the amount of $NH_3$ including ammonium ions or deamide-NAD in a specimen is measured.

②lactate dehydrogenase ( EC 1.1.1.27 ):

$$pyruvate + NADH + H^+ \rightarrow L\text{-lactate} + NAD$$

The decrease of $A_{340}$ according to an oxidation of NADH is measured in the presence of excess lactate dehydrogenase and NADH.

As hereinabove illustrated, in the present invention not only the reaction mixture containing ATP or $NH_3$, which is consumed or generated in the illustrated enzymatic reaction system, but also the reaction mixture for measuring the enzymatic activity which is used in the enzymatic reaction system, consumed substrate or generated product, can be used as a specimen to be assayed.

In these enzymatic reaction systems, ATP or $NH_3$ is assayed to determine the enzymatic activity in the said enzymatic reaction or measure the amount of any one of the components thereof. A substance other than the components to be assayed is added at a constant rate as a reagent. The amount of the specimen or reagent can be varied depending on the objects and conditions.

Examples of oxidation-reduction systems with coenzyme NAD are reaction system constituting dehydrogenase ( $E_1$ ) which consume NAD to generate NADH and its substrate( $S_1$ ), or dehydrogenase ( $E_1$ ) with coenzyme NAD or NADP and its substrate ( $S_1$ ). The source of the dehydrogenase is not limited and at least this enzyme reacts with specific substrates and consumes coenzyme NAD to form NADH.

Examples of these enzymes and substrates are mentioned in the " Enzyme Handbook". Examples are:

lactate dehydrogenase ( EC 1.1.1.27 ) and L-lactate,
glycerol dehydrogenase ( EC 1.1.1.6 ) and glycerol,
glycerol-3-phosphate dehydrogenase ( EC 1.1.1.8 ) and glycerol-3-phosphate,
glucose dehydrogenase ( EC 1.1.47 ) and glucose,
malate dehydrogenase ( EC 1.1.1.37 ) and L-malate,
glutamate dehydrogenase ( EC 1.4.1.2 ) and L-glutamate,
3- α-hydroxysteroid dehydrogenase ( EC 1.1.1.50 ) and 3- α-hydroxysteroid.

The amount of the enzyme used in these oxidation-reduction reactions varies depending on the enzyme activity, the kind of substrate and the ratio of coenzyme cycling. The substrate should be in molar excess as compared with the cycling coenzyme, because one molar ratio of substrate is consumed per cycle.The amount of the substrate is determined by the number of cycles per hour and the reaction time. The concentration of the substrate is preferably determined to attain a maximum reaction rate of oxido-reductase, and is from 0.1 mM to 100 mM.

The reaction system for coenzyme NADH is a reaction system of functional substances ( $E_2$ ) , which at least consumes NADH and generates NAD, and its substrate ( $S_2$ ). Examples are a reaction system with oxidoreductase, which at least consumes NADH and generates NAD, and its substrate; and a reaction system consisting of an electron-transfer agent and a tetrazolium salt.

Co enzyme cycling reaction system :

(a) oxidation-reduction reaction system with coenzyme NAD;

$$NAD + S_1 \xrightarrow[E_1]{} NADH + P_1$$

(b) transfer reaction system with coenzyme NADH;

$$NADH + S_2 \xrightarrow[E_2]{} NAD + P_2$$

wherein :

$NH_3$ : compound containing monovalent ammonium ions,
$E_1$ : dehydrogenase which catalyzes a reaction consuming the substrates NAD and $S_1$, and generating NADH

and $P_1$,

$E_2$ : active substance which catalyzes a reaction consuming NADH and $S_2$, and generating NAD and $P_2$,

$S_1$ : reduced substrate in $E_1$,

$S_2$ : oxidized substrate in $E_2$,

$P_1$ : oxidation product of $S_1$,

$P_2$ : reduction product of $S_2$.

A reaction utilizing $NH_3$ is illustrated as follows:

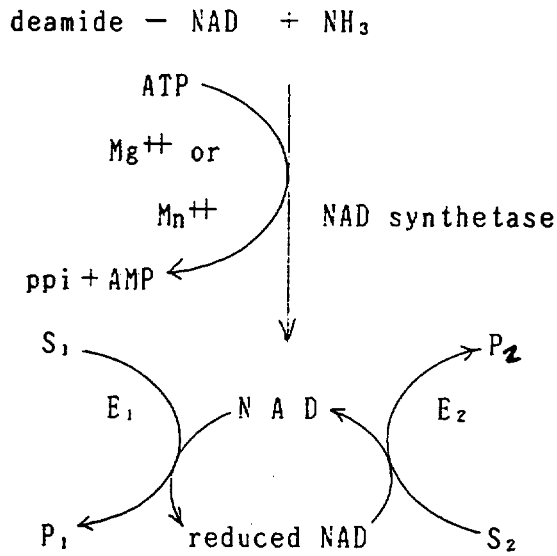


Examples of the oxidoreductase are a dehydrogenase which catalyzes, with at least coenzyme NADH, a reaction of an excess amount of specific substrate ( $S_2$ ) to form NAD and reduced substrate ( $P_2$ ) of $S_2$ ; or NADH: ( acceptor ) oxidoreductase wherein at least NADH is the coenzyme and the acceptor is cytochrome, a disulfide coumpound, quinone or its analogues. These enzymes, substrates and acceptors are mentioned in " Enzyme Handbook ".

Examples of dehydrogenase and its substrate are lactate dehydrogenase ( EC 1.1.1. 27 ) and pyruvate, alcohol dehydrogenase ( EC 1.1.1 ) and acetaldehyde, and glycerol dehydrogenase ( EC 1.1.1.6 ) and dihydroxyacetone,

Examples of NADH : ( acceptor ) oxidoreductase are cytochrome bs reductase ( EC 1.6.2.2 ) and diaphorase.

Examples of acceptors are methylene blue, flavins, quinones and 2,6-dichlorophenol indophenol.

The combination of NADH: ( acceptor ) oxidoreductase and acceptor is not limited to an enzyme with coenzyme NADH and an electron acceptor, and is preferably diaphorase ( EC 1.6.4.3 ) and a tetrazolium salt, and methylene blue, NAD dehydrogenase ( EC 1.6.99.3 ) and cytochrome c. The concentration thereof is usually from 0.05 to 100 U/m ℓ. The concentration of the tetrazolium salt depends upon the solubility of the tetrazolium salt and the ultimately generated formazane, and is generally from 1 to 100 μg per ml of reagent.

Examples of electron transfer agents are substances which oxidize NADH to NAD without having a detrimental effect on coenzyme cycling, for example phenazine methosulfate, meldola blue or pyrocyanine. The concentration thereof depends on the cycling ratio and is from 5 μg to 0.5 mg per ml of the reaction mixture.

The above cycling reaction is usually carried out at from room temperature to 37 °C, preferably from 30 to 37 °C. The reaction time is not limited but is usually at least one minute, preferably at least 5 minutes. The reaction can be terminated by adding an acid such as hydrochloric acid or phosphoric acid.

After terminating the cycling reaction, the amount of a substance consumed or generated in the cycling reaction is measured. Examples of the substance are the reduction product ( $P_1$ ) from the reduced substrate ( $S_1$ ) of $E_1$, or the reduced product ( $P_2$ ) from the oxidized substrate ( $S_2$ ) of $E_2$ as a generated component, and the reduced substrate ( $S_1$ ) of $E_1$ or the oxidized substrate ( $S_2$ ) of $E_2$ as a consumed component. One of components $P_1$, $P_2$, $S_1$, or $S_2$ is measured Most preferably, a product which is colourless as a substrate and coloured or fluorescent as a product is colourimetrically measured by absorbency changes. For example, formzane generated from a tetrazolium substrate ($S_2$ ) is reduced to form a reduced product ( $P_2$ ) which is measured colourimetrically. Furthermore, when flavins or quinones are used as a substrate ( $S_2$ ), the amount of the substrate ( $S_2$ ) consumed is preferably measure by colourimetry.

In the above reaction, a surface active agent is preferably added to prevent the precipitation of formazane from

the tetrazolium salt. Examples of surface active agents are non-ionic surface active agents such as Triton X-100 ( iso-octyl phenoxy polyethyloxy ethanol, Rohm & Haas Co, USA ) or Adekatol SO-120 ( ethoxylate of a secondary alcohol, Asahi Denka Kogyo Co, Japan ). The concentration thereof is from 0.01 to 3 %. Adding a surface active agent provides increased measurement sensitivity and stability of the formazane pigment.

The colourimetric assay of the generated formazane pigment can be performed by measuring the optical densitiy ( OD ) at its specific adsorption wavelength, such as at from 500 to 550 nm.

The method of the present invention can late the form of, for example, an end-point assay method, a rate assay method or a dry-chemical method ( film method or immobilized solid ).

The method of the present invention is useful for the assay of any one of ATP, deamide-NAD or ammonia including ammonia in the form of ammonium ions. Ammonia including ammonium ions can be assayed without affecting the amino acids in a specimen. Furthermore, the NAD synthetase of the present invention is heat stable.

The following Examples further illustrate the present invention·

Example 1

A liquid medium ( pH 7.6, 40 l ) consisting of 1% peptone 0.5% glucose, 0.05% Nacℓ and 0.05% $MgSO_4 \cdot 7H_2O$ in a 50 ℓ jar fermenter was sterilized at 120°C for 20 minutes. A previously-cultured Bacillus stearothermophilus H-804 seed-culture medium of the same composition ( 200 ml ) was inoculated therein and the mixture was cultured at 60°C for 10 hours with an aeration of 40 ℓ/min and an agitation of 150 r.p.m. After cultivation, the cells were collected by centrifugation, suspended in 10 mM tris-HCℓ ( pH 8.0, 500 mℓ) containing 0.1 % lysozyme, and the medium was incubated at 37°C for 30 mins to lyse the cells. The lysed solution was centrifuged at 5,000 r.p.m. for 10 mins to obtain a supernatant solution ( 450 mℓ). Ammonium sulfate was added thereto to fractionate the solution ( 0.5 to 0.71 saturation ) and the obtained preciptiate , dissolved in 10 mM Tris-HC ℓ buffer ( 50m ℓ, 21 U), was dialyzed against the same buffer ( 5ℓ ) The preciptated insolubles were removed by centrifugation ( 15,000 r.p.m., 10 min ) The supernatant solution ( 20 U ) was charged on a column ( 2.5 × 5 cm ) of DEAE-Sepharose CL-6B buffered with 10 mM Tris-HCℓ buffer ( pH 8.0 ) and eluted with a gradient of 0 to 0.5 M NaCℓ. The fractions eluting with 0.25 to 0.3 M NaCℓ were collected ( 80 mℓ, 16.5 U), concentrated by ultra-filtration using a CF-25 membrane ( Amicon Co. centriflow membrane cone ), chromatographed with Sephadex G-100 ( 3.6 × 80 cm ) and the active fractions collected to obtain the purified solution ( 5 mℓ, 14 U ).

Example 2

Assay of ATP in a specimen:

50 mM Tris-HCℓ buffer pH 8.0
20 mM KC ℓ
5 mM $MgCℓ_2$
0.05 % bovine serum albumin
1 mM deamide-NAD
50 mM $( NH_4 )_2SO_4$,
100 mU/m ℓ the present NAD synthetase

Reaction medium II :

50 mM Tris-HCℓ buffer pH 8.0
10 U diaphorase/m ℓ ( Toyo Jozo, Bacillus )
3 % ethanol
10 U alcohol dehydrogenase/m ℓ ( Toyobo, yeast )
0.025 % NTB
0.1 % Triton X-100
15 mM EDTA

Reaction mixture I ( 0.3 mℓ) in a test tube was pre -incubated at 37°C, and 0, 10, 20, 30 and 40 μM ATP solutions ( 5 μℓ each ) were respectively added thereto; each was then incubated at 37°C for 10 minutes. Reaction medium II ( 0.7 ml ) was added thereto, and each was incubated at 37°C for exactly 5 minutes, whereupon the reaction was stopped by adding 0.1 NHC ℓ ( 2.0 ml ) and the absorbency was measured at 550 nm. Good linearity was obtained.

Example 3

Measurement of ammonium ion :

50 mM ( $NH_4$ )$_2SO_4$ in reaction medium I in Example 2 was replaced by 5 mM ATP to prepare the reaction medium. Various concentrations of ammonium sulfate ( 0, 5, 10, 15 and 20μM, 5μ ℓ ), were added thereto and the media were treated the same way as in Example 2. Good linearity was obtained.

Example 4

Assay of creatinine :

Reaction medium I :

50 mM Tris-HC ℓ buffer pH 8.0
10 mM KC ℓ
5 mM $MgCl_2$
1 mM ATP
0.05 % bovine serum albumin
1 mM deamide-NAD
20 U/m ℓ creatinine deaminase ( KODAK )
100 mU/ml the present NAD synthetase

Reaction mixture I ( 0.3 ml ) in a test tube was pre-incubated at 37 °C, and 1, 2, 3 and 4 mg/dℓ creatinine solutions ( 10 μ ℓ each ) were respectively added thereto; They were then treated the same way as in Example 2. Good linearity was obtained.

Example 5

Assay of ammonium ion :

Reaction medium 1 :

50 mM Tris-HC ℓ buffer pH 8.0
10 mM KC ℓ
5 mM $MgCl_2$
1 mM ATP
0.05 % bovine serum albumin
1 mM deamide-NAD
100 mU/ml the present NAD synthetase

Reaction mixture 1 ( 0.1 ml ) in a test tube was pre-incubated at 37 C, and 0, 2.5, 5.0, 7.5 and 10.0 μM ( $NH_4$ )$_2SO_4$ solutions (10 μ ℓ each ) were respectively added thereto; each was then incubated at 37°C for 20 minutes and the absorbency was measured at 340 nm. Good linearity was obtained.

Example 6

Assay of generated ATP :

Reaction medium I :

50 mM Tris-HC ℓ buffer pH 8.0
10 mM KC ℓ
5 mM $MgCl_2$
1 mM ATP
0.05 % bovine serum albumin
0.05 % Triton X-100
1 mM deamide-NAD
10 U/m ℓ myokinase kinase ( Sigma, Bacillus )
10 U/mℓ pyruvate kinase ( Sigma, Bacillus )
10 mM phosphoenol pyruvate
100 mU/m ℓ the present NAD synthetase

Reaction medium II:

50 mM phosphate buffer pH 8.0
100 mU/m ℓ pyruvate oxidase ( Sigma, Pediococcus )
100 mU/m ℓ peroxidase ( Sigma )
0.2 % phenol
0.3 % 4-aminoantipyrine

Reaction mixture I ( 1 ml ) in a test tube was pre-incubated at 37 °C, and 0, 0.5, 1.5 and 2.0 mM ( $NH_4$ )$_2SO_4$ solutions ( 10 µ ℓ each ) were respectively added thereto; each was then incubated at 37 °C for 30 minutes. Reaction medium II ( 0.1 ml ) was added thereto, and each incubated at 37°C for 30 minutes. The absorbency was measured at 550 nm. Good linearity was obtained.

Example 7

Assay of diamide-NAD :
Reaction medium 1 :

50 mM Tris-HC ℓ buffer pH 8.0
10 mM KC ℓ
5 mM $MgCl_2$
0.05 % bovine serum albumin
50 mM ( $NH_4$ )$_2OS_4$
1 mM ATP
100 mU/m ℓ the present NAD synthetase
3 % ethanol
10 U alcohol dehydrogenase
0.1 % Triton X-100

Reaction mixture ( 1 ml ) in a test tube was pre-incubated at 37 °C, and 0, 2.5, 5 and 10 mM diamideNAD solutions ( 10µ ℓ each ) were respectively added thereto; each was then incubated at 37°C for 30 minutes and the absorbency was measured at 340 nm. Good linearity was obtained.

Example 8

Optimum concentration of metallic ion :
Reaction medium I :

50 mM Tris-HC ℓ buffer pH 8.0
10 mM KC ℓ
2 mM ATP
0.05 % bovine serum albumin
0.5 mM deamide-NAD
25 mM ( $NH_4$ )$_2SO_4$

Reaction medium II:

50 mM Tris-HC ℓ buffer pH 8.0
10 U diaphorase/m ℓ ( Toyo Jozo Co, Bacillus )
3 % ethanol
10 U alcohol dehydrogenase/mℓ (Toyobo Co, yeast)
0.025 % NTB
0.1 % Triton X-100
10mM EDTA

To a reaction medium I in a test tube were added 0.25, 0.50, 0.75, 1, 2, 3 and 4 mM $MgCℓ_2$ and 0.25, 0.50, 0.75, 1, 2, 3 and 4 mM $MnCℓ_2$, respectively; each was then treated, after adding the present NAD synthetase solution, according to an assay method as previously illustrated.

Optimum concentration of $MnC\ell_2$ is at 3mM.

## Claims

1. A method of assaying a specimen for ATP, deamide-NAD, ammonia including ammonia in the form of an ammonium ion or a component of a reaction system in which ATP, deamide-NAD or ammonia including ammonia in the form of an ammonium ion is generated or consumed, which method comprises:

   (i) incubating the specimen with a NAD synthetase having the following properties:

   (1) Action:
   at least catalyses in the presence of $Mg^{++}$ or $Mn^{++}$ ion the reaction (a):

$$\text{(a)}\quad ATP + \text{deamide-NAD} + NH_3 \xrightleftharpoons{Mg^{++}\text{ or } Mn^{++}} AMP + PPi + NAD$$

   and does not catalyse in the presence of $Mg^{++}$ ion the reaction (b):

$$\text{(b)}\quad ATP + \text{deamide-NAD} + \text{L-Gln} + H_2O \overset{Mg^{++}}{\rightleftarrows} AMP + PPi + NAD + \text{L-Glu}$$

   (2) Substrate specificity:
   utilises ammonia, including ammonia in the form of an ammonium ion, as a substrate but does not utilise glutamine or asparagine as a substrate;
   in the presence of $Mg^{++}$ or $Mn^{++}$ ions and the two of ATP, deamide-NAD and ammonia which may be in the form of an ammonium ion which are not being assayed for or which are not generated or consumed by the said reaction system, to generate thereby NAD;

   (ii) subjecting the thus-generated NAD to a coenzyme cycling reaction comprising reducing said generated NAD to reduced NAD and oxidizing the thus-generated reduced NAD to NAD; and
   (iii) measuring a compound which is consumed or generated in said cycling reaction.

2. A method according to claim 1, wherein the NAD synthetase has the following properties:

   (1) optimum pH : pH 8.5. - 10.0
   (2) pH-stability : pH 7.5 - 9.0 (at 60°, 15 min)
   (3) optimum temperature : approx. 60°C and
   (4) heat stability : below 60°C (at pH 8.0, 15 min).

3. A method according to claim 1 or 2, wherein the NAD synthetase has the following properties:

   (1) isoelectric point: approx. pH 5.3 and
   (2) molecular weight : 50,000 ± 5,000 (molecular sieve using polyvinyl gel).

4. A method according to any one of claims 1 to 3, wherein the NAD synthetase is obtainable from Bacillus stearothermophilus H-804 (FERM BP-1408).

5. A method according to any one of claims 1 to 4, wherein said ATP is generated by an enzymatic reaction selected from:

   (1) creatine kinase:

$$\text{creatine phospate} + \text{ADP} \xrightarrow[\text{Mg}^{++}]{\text{reducing agent}} \text{creatine} + \text{APT}$$

reducing agent: β-mercapto ethanol, reduced glutathione, cysteine, N-acetyl-cysteine or dithiothreitol.

(2) pyruvate kinase:

$$\text{phosphoenol pyruvate} + \text{ADP} \xrightarrow{\text{Mn}^{++}\text{ or Mg}^{++},\ \text{K}^{+}} \text{pyruvate} + \text{ATP}$$

(3) acetate kinase:

$$\text{acetylphosphate} + \text{ADP} \xrightarrow{\text{Mg}^{++}\text{ or Mn}^{++}} \text{acetate} + \text{ATP}$$

(4) carbamate kinase:

$$\text{carbamylphosphate} + \text{ADP} \xrightarrow{\text{Mg}^{++}} \text{NH}_3 + \text{CO}_2 + \text{ATP}$$

(5) aspartate kinase:

$$\text{4-phospho-L-aspartate} + \text{ADP} \xrightarrow{\text{Mg}^{++}} \text{L-aspartate} + \text{ATP}$$

(6) phosphoglycerate kinase:

$$\text{1,3-diphospho-D-glycerate} + \text{ADP} \xrightarrow{\text{Mg}^{++}\text{ or Mn}^{++}} \text{3-phospho-D-glycerate} + \text{ATP; and}$$

(7) arginine kinase:

$$\text{arginine phosphate} + \text{ADP} \xrightarrow{\text{Mg}^{++}\text{ or Mn}^{++}} \text{L-arginine} + \text{ATP}$$

**6.** A method according to any one of claims 1 to 4, wherein said ammonia or ammonium ion is ammonium chloride, aqueous ammonia, ammonium sulfate, ammonium nitrate, ammonium acetate or ammonium citrate or is generated or consumed by an enzymatic reaction selected from:

(1) nicotine amidase:

$$\text{nicotine amide} + H_2O \rightarrow \text{nicotinate} + NH_3 + H^+$$

(2) glutaminyl-peptide glutaminase:

$$\text{L-glutaminyl-peptide} + H_2O \rightarrow \text{L-glutamyl-peptide} + NH_3$$

(3) arginine deaminase:

$$\text{L-arginine} + H_2O \rightarrow \text{citrulline} + NH_3 + H^+$$

(4) guanine deaminase:

$$\text{guanine} + H_2O \rightarrow \text{xanthine} + NH_3 + H^+$$

(5) adenosine deamidase:

$$\text{adenosine} + H_2O \rightarrow \text{inosine} + NH_3 + H^+$$

(6) creatinine deamidase:

$$\text{creatinine} + H_2O \rightarrow \text{N-methylhydantoin} + NH_3 + H^+$$

(7) threonine dehydratase:

$$\text{L-threonine} + H_2O \rightarrow \text{2-oxobutyrate} + CO_2 + NH_3 + H^+$$

(8) aspartate ammonia-lyase:

$$\text{L-aspartate} \rightarrow \text{fumarate} + NH_3 + H^+$$

(9) L-methionine-$\gamma$-lyase:

$$\text{L-methionine} + H_2O \rightarrow \text{2-oxobutyrate} + CH_3SH + NH_3 + H^+;$$

and
(10) methylamino-glutamate methyl transferase:

$$\text{N-methylglutamate} + NH_3 + H^+ \rightleftarrows \text{glutamate} + \text{methylamine}.$$

**7.** A method according to any one of claims 1 to 6, wherein said generated NAD is reduced to said reduced NAD in an enzymatic reaction system comprising dehydrogenase, which consumes NAD and generates reduced NAD,

and a substrate thereof.

**8.** A method according to claim 7, wherein said dehydrogenase and substrate thereof are selected from:

lactate dehydrogenase and L-lactate;
alcohol dehydrogenase and ethanol;
glycerol-3-phosphate dehydrogenase and glycerol-3-phosphate;
glucose dehydrogenase and glucose;
malate dehydrogenase and L-malate;
glutamate dehydrogenase and L-glutamate; and
3-α-hydroxysteroid dehydrogenase and 3-α-hydroxysteroid.

**9.** A method according to any one of claims 1 to 8, wherein said reduced NAD is oxidised to NAD in a reaction system comprising oxido-reductase, which consumes reduced NAD and generates oxidised NAD, and a substrate thereof.

**10.** A method according to claim 9, wherein said oxido-reductase and substrate thereof are selected from:

lactate dehydrogenase and pyruvate;
alcohol dehydrogenase and acetaldehyde;
glycerol dehydrogenase and dihydroxyacetone;
glycerol-3-phosphate dehydrogenase and
dihydroxyacetone phosphate;
malate dehydrogenase and oxaloacetate; and
3-α-hydroxysteroid dehydrogenase and 3-ketosteroid.

**11.** A method according to claim 9, wherein said oxido-reductase and substrate thereof is reduced NAD: (acceptor) oxido-reductase and an acceptor thereof.

**12.** A method according to claim 11, wherein the reduced NAD: (acceptor) oxido-reductase and an acceptor thereof are reduced NAD dehydrogenase and a member selected from flavins, quinones, 2,6-dichlorophenol indophenol, ferricyanide salt, tetrazolium salt, cytochrome c and oxygen.

**13.** A method according to claim 9, wherein said oxido-reductase and substrate thereof are diaphorase tetrazolium salt and resazurium.

**14.** A method according to any one of claims 1 to 8, wherein said reduced NAD is oxidized to NAD by means of an electron-transfer agent and tetrazolium.

**15.** A method according to claim 14, wherein said electron-transfer agent is phenazine methosulfate, meldola blue or pyrocyanine.

**16.** A method according to any one of claims 1 to 4, wherein AMP is measured by an enzymatic reaction system selected from:

(1) adenylate kinase:

$$AMP + ATP \rightleftarrows ADP + ADP$$

pyruvate kinase:

$$ADP + \text{phosphoenol pyruvate} \rightleftarrows ATP + \text{pyruvate}$$ pyruvate oxidase:

$$\text{pyruvate} + Pi + O_2 + H_2O \rightarrow \text{acetylphosphate} + CO_2 + H_20_2$$

(generated $H_2O_2$ is measured); and

(2) adenylate kinase:

$$AMP + ATP \rightleftarrows ADP + ADP$$

pyruvate kinase:

$$ADP + \text{phosphoenol pyruvate} \rightleftarrows ATP + \text{pyruvate lactate dehydrogenase:}$$

$$\text{pyruvate} + NADH + H^{+} \rightarrow \text{L-lactate} + NAD$$

(decreased NADH is measured)

**17.** A method according to any one of claims 1 to 16, wherein said cycling reaction is carried out in the presence of a surface active agent.

**18.** A method according to claim 17, wherein said surface active agent is a non-ionic surface active agent.

**Patentansprüche**

**1.** Verfahren zum Test einer Probe auf ATP, Deamid-NAD, Ammoniak einschließlich Ammoniak in Form eines Ammoniumions oder eine Komponente eines Reaktionssystems, bei dem ATP, Deamid-NAD oder Ammoniak einschließlich Ammoniak in Form eines Ammoniumions erzeugt oder verbraucht wird, dadurch gekennzeichnet, daß man

(i) die Probe mit einer NAD-Synthetase mit den folgenden Eigenschaften

(1) Wirkung:
katalysiert mindestens in Gegenwart von $Mg^{++}$- oder $Mn^{++}$-Ionen die Reaktion

$$\text{(a) } ATP + \text{Deamid-NAD} + NH_3 \xrightleftharpoons{Mg^{++} \text{ oder } Mn^{++}} AMP + PPi + NAD$$

und katalysiert in Gegewart von $Mg^{++}$-Ionen nicht die Reaktion (b):

$$\text{(b) } ATP + \text{Deamid-NAD} + \text{L-Gln} + H_2O \xrightleftharpoons{Mg^{++}} AMP + PPi + NAD + \text{L-Glu}$$

(2) Substratspezifität:
verwertet Ammoniak einschließlich Ammoniak in Form eines Ammoniumions als Substrat, aber verwertet nicht Glutamin oder Asparagin als Substrat;
in Gegenwart von $Mg^{++}$- oder $Mn^{++}$-Ionen und zwei der Verbindungen ATP, Deamid-NAD und Ammoniak, das in Form eines Ammoniumions vorliegen kann, die nicht getestet werden und die von dem Reaktionssystem nicht erzeugt oder verbraucht werden, inkubiert, wodurch NAD erzeugt wird:

(ii) das so erzeugte NAD einer Coenzymcyclisierungsreaktion, umfassend die Reduktion des erzeugten NADs zu reduziertem NAD und die Oxidation des so erzeugten reduzierten NADs zu NAD, unterwirft und

(iii) eine Verbindung, die bei der Cyclisierungsreaktion verbraucht oder erzeugt wird, mißt.

**2.** Verfahren nach Anspruch 1, dadurch gekennzeichnet, daß die NAD-Synthetase die folgenden Eigenschaften besitzt:

(1) pH-Optimum: pH 8,5 - 10,0
(2) pH-Stabilität: pH 7,5 - 9,0 (bei 60 °C, 15 min)
(3) Temperaturoptimum: etwa 60 °C und
(4) Wärmestabilität: unter 60 °C (bei pH 8,0; 15 min).

**3.** Verfahren nach Anspruch 1 oder 2, dadurch gekennzeichnet, daß die NAD-Synthetase die folgenden Eigenschaften besitzt:

(1) Isoelektrischer Punkt: etwa pH 5,3 und
(2) Molekulargewicht: 50.000 ± 5.000 (Molekularsieb unter Verwendung eines Polyvinylgels).

**4.** Verfahren nach einem der Ansprüche 1 bis 3, dadurch gekennzeichnet, daß die NAD-Synthetase von Bacillus stearothermophilus H-804 (FERM BP-1408) erhältlich ist.

**5.** Verfahren nach einem der Ansprüche 1 bis 4, dadurch gekennzeichnet, daß ATP durch eine enzymatische Reaktion ausgewählt aus:

(1) Creatinkinase:

$$\text{Creatinphosphat} + \text{ADP} \xrightarrow[Mg^{++}]{\text{Reduktionsmittel}} \text{Creatin} + \text{ATP}$$

Reduktionsmittel: β-Mercaptoethanol, reduziertes Glutathion, Cystein, N-Acetylcystein oder Dithiothreit

(2) Pyruvatkinase:

$$\text{Phosphoenolpyruvat} + \text{ADP} \xrightarrow{Mn^{++} \text{ oder } Mg^{++},\ K^{+}} \text{Pyruvat} + \text{ATP}$$

(3) Acetatkinase:

$$\text{Acetylphosphat} + \text{ADP} \xrightarrow{Mg^{++} \text{ oder } Mn^{++}} \text{Acetat} + \text{ATP}$$

(4) Carbamatkinase:

$$\text{Carbamylphosphat} + \text{ADP} \xrightarrow{Mg^{++}} NH_3 + CO_2 + \text{ATP}$$

(5) Aspartatkinase:

$$\text{4-Phospho-L-aspartat} + \text{ADP} \xrightarrow{Mg^{++}} \text{L-Aspartat} + \text{ATP}$$

(6) Phosphoglyceratkinase:

$$\text{1,3-Diphospho-D-glycerat} + \text{ADP} \xrightarrow{Mg^{++} \text{ oder } Mn^{++}} \text{3-Phospho-D-glycerat} + \text{ATP}; \text{ und}$$

(7) Argininkinase:

$$\text{Argininphosphat} + \text{ADP} \xrightarrow{Mg^{++} \text{ oder } Mn^{++}} \text{L-Arginin} + \text{ATP}$$

erzeugt wird.

**6.** Verfahren nach einem der Ansprüche 1 bis 4, dadurch gekennzeichnet, daß der Ammoniak oder das Ammoniumion Ammoniumchlorid, wäßriger Ammoniak, Ammoniumsulfat, Ammoniumnitrat, Ammoniumacetat oder Ammoniumcitrat ist oder durch eine enzymatische Reaktion ausgewählt aus:

(1) Nicotinamidase:

$$\text{Nicotinamid} + H_2O \rightarrow \text{Nicotinat} + NH_3 + H^+$$

(2) Glutaminylpeptidglutaminase:

$$\text{L-Glutaminylpeptid} + H_2O \rightarrow \text{L-Glutamylpeptid} + NH_3$$

(3) Arginindeaminase:

$$\text{L-Arginin} + H_2O \rightarrow \text{Citrullin} + NH_3 + H^+$$

(4) Guanindeaminase:

$$\text{Guanin} + H_2O \rightarrow \text{Xanthin} + NH_3 + H^+$$

(5) Adenosindeamidase:

$$\text{Adenosin} + H_2O \rightarrow \text{Inosin} + NH_3 + H^+$$

(6) Creatinindeamidase:

$$\text{Creatinin} + H_2O \rightarrow \text{N-Methylhydantoin} + NH_3 + H^+$$

(7) Threonindehydratase:

$$\text{L-Threonin} + H_2O \rightarrow \text{2-Oxobutyrat} + CO_2 + NH_3 + H^+$$

(8) Aspartat-Ammoniaklyase:

$$\text{L-Aspartat} \rightarrow \text{Fumarat} + NH_3 + H^+$$

(9) L-Methionin-$\gamma$-lyase:

$$\text{L-Methionin} + H_2O \rightarrow \text{2-Oxobutyrat} + CH_3SH + NH_3 + H^+$$

und

(10) Methylaminoglutamatmethyltransferase:

$$\text{N-Methylglutamat} + NH_3 + H^+ \rightleftarrows \text{Glutamat} + \text{Methylamin}$$

erzeugt oder verbraucht wird.

**7.** Verfahren nach einem der Ansprüche 1 bis 6, dadurch gekennzeichnet, daß das erzeugte NAD zu reduziertem NAD in einem enzymatischen Reaktionssystem, umfassend Dehydrogenase, die NAD verbraucht und reduziertes NAD erzeugt, und einem Substrat davon reduziert wird

**8.** Verfahren nach Anspruch 7, dadurch gekennzeichnet, daß die Dehydrogenase und das Substrat davon aus:

Lactatdehydrogenase und L-Lactat;
Alkoholdehydrogenase und Ethanol;
Glycerin-3-phosphatdehydrogenase und Glycerin-3-phosphat;
Glucosedehydrogenase und Glucose;
Malatdehydrogenase und L-Malat;
Glutamatdehydrogenase und L-Glutamat; und
3-$\alpha$-Hydroxysteroiddehydrogenase und 3-$\alpha$-Hydroxysteroid

ausgewählt werden.

**9.** Verfahren nach einem der Ansprüche 1 bis 8, dadurch gekennzeichnet, daß das reduzierte NAD zu NAD in einem Reaktionssystem, umfassend eine Oxidoreduktase, die reduziertes NAD verbraucht und oxidiertes NAD erzeugt, und einem Substrat davon, oxidiert wird.

**10.** Verfahren nach Anspruch 9, dadurch gekennzeichnet, daß die Oxidoreduktase und das Substrat davon aus:

Laktatdehydrogenase und Pyruvat;
Alkoholdehydrogenase und Acetaldehyd;
Glycerindehydrogenase und Dihydroxyaceton;
Glycerin-3-phosphatdehydrogenase und Dihydroxyacetonphosphat;
Malatdehydrogenase und Oxalacetat; und
3-a-Hydroxysteroiddehydrogenase und 3-Ketosteroid

ausgewählt werden.

**11.** Verfahren nach Anspruch 9, dadurch gekennzeichnet, daß die Oxidoreduktase und das Substrat davon reduziertes NAD: (Akzeptor)-Oxidoreduktase und ein Akzeptor davon sind.

**12.** Verfahren nach Anspruch 11, dadurch gekennzeichnet, daß die reduzierte NAD: (Akzeptor)-Oxidoreduktase und ein Akzeptor davon reduzierte NAD-Dehydrogenase und eine Verbindung, ausgewählt aus Flavinen, Chinonen, 2,6-Dichlorophenolindophenol, Ferricyanidsalz, Tetrazoliumsalz, Cytochrom c und Sauerstoff sind.

**13.** Verfahren nach Anspruch 9, dadurch gekennzeichnet, daß die Oxidoreduktase und das Substrat davon Diaphorasetetrazoliumsalz und Resazurium sind.

**14.** Verfahren nach einem der Ansprüche 1 bis 8, dadurch gekennzeichnet, daß das reduzierte NAD zu NAD mit einem Elektronentransfermittel und Tetrazolium oxidiert wird.

**15.** Verfahren nach Anspruch 14, dadurch gekennzeichnet, daß das Elektronentransfermittel Phenazinmethosulfat, Meldola Blau oder Pyrocyanin ist.

**16.** Verfahren nach einem der Ansprüche 1 bis 4, dadurch gekennzeichnet, daß AMP in einem enzymatischen Reaktionssystem ausgewählt aus:

(1) Adenylatkinase:

$$AMP + ATP \rightleftarrows ADP + ADP$$

Pyruvatkinase:

$$ADP + Phosphoenolpyruvat \rightleftarrows ATP + Pyruvat$$

Pyruvatoxidase:

$$Pyruvat + Pi + O_2 + H_2O \rightarrow Acetylphosphat + CO_2 + H_2O_2$$

(das erzeugte $H_2O_2$ wird gemessen);
und

(2) Adenylatkinase:

$$AMP + ATP \rightleftarrows ADP + ADP$$

Pyruvatkinase:

$$ADP + Phosphoenolpyruvat \rightleftarrows ATP + Pyruvat$$

Lactatdehydrogenase:

$$\text{Pyruvat} + \text{NADH} + H^+ \rightarrow \text{L-Lactat} + \text{NAD}$$

(die NADH-Abnahme wird gemessen)

gemessen wird.

**17.** Verfahren nach einem der Ansprüche 1 bis 16, dadurch gekennzeichnet, daß die Cyclisierungsreaktion in Gegenwart eines grenzflächenaktiven Mittels durchgeführt wird.

**18.** Verfahren nach Anspruch 17, dadurch gekennzeichnet, daß das grenzflächenaktive Mittel ein nicht-ionisches grenzflächenaktives Mittel ist.

## Revendications

**1.** Méthode de dosage d'un spécimen pour ATP, désamide-NAD, ammoniaque comprenant l'ammoniaque sous la forme d'un ion ammonium ou d'un composant d'un système réactionnel où ATP, désamide-NAD ou l'ammoniaque comprenant l'ammoniaque sous la forme d'un ion ammonium est généré ou consommé, laquelle méthode comprend :

(i) l'incubation du spécimen avec une NAD synthétase ayant les propriétés suivantes :

(1) Action :
catalyse au moins en présence de l'ion $Mg^{++}$ ou $Mn^{++}$ la réaction (a) :

$$\text{(a)} \quad \text{ATP} + \text{désamide-NAD} + NH_3 \xrightleftharpoons{Mg^{++} \text{ ou } Mn^{++}} \text{AMP} + \text{PPi} + \text{NAD}$$

et ne catalyse pas en présence de l'ion $Mg^{++}$ la réaction (b):

$$\text{(b)} \quad \text{ATP} + \text{désamide-NAD} + \text{L-Gln} + H^2O \xrightarrow{Mg^{++}} \text{AMP} + \text{PPi} + \text{NAD} + \text{L-Glu}$$

(2) Spécificité du substrat :
utilise l'ammoniaque, comprenant l'ammoniaque sous la forme d'un ion ammonium, en tant que substrat, mais n'utilise pas la glutamine ni l'asparigine comme substrat ; en présence des ions $Mg^{++}$ ou $Mn^{++}$ et de deux de ATP, désamide-NAD ou ammoniaque qui peut être sous la forme d'un ion ammonium, qui ne sont pas dosés ou qui ne sont générés ni consommés par le système réactionnel, pour ainsi produire NAD ;

(ii) la soumission de NAD ainsi produit a une réaction de cyclage de coenzyme comprenant la réduction dudit NAD généré en NAD réduit et l'oxydation de NAD réduit ainsi généré en NAD ; et
(iii) la mesure d'un composé qui est consommé généré dans ladite réaction de cyclage .

**2.** Méthode selon la revendication 1, où la NAD synthétase a les propriétés suivantes :

(1) pH optimum : pH 8,5-10,0
(2) stabilité pH : pH 7,5-9,0 (à 60°, 15 minutes)
(3) température optimale : environ 60°C et
(4) stabilité à la chaleur : moins de 60°C (à pH 8,0, 15 minutes).

**3.** Méthode selon la revendication 1 ou 2, où la NAD synthétase a les propriétés suivantes :

(1) point isoélectrique : environ pH 5,3 et
(2) poids moléculaire : 50 000 ± 5000 (tamis moléculaire en utilisant du gel de polyvinyle).

**4.** Méthode selon l'une quelconque des revendications 1 à 3, où la NAD synthétase peut être obtenue de Bacillus stearothermophilus H-804 (FERM BP-1408).

**5.** Méthode selon l'une quelconque des revendications 1 à 4, où ledit ATP est généré par une réaction enzymatique sélectionnée parmi :

(1) créatine kinase :

$$\text{créatine phosphate} + \text{ADP} \xrightarrow[\text{Mg}^{++}]{\text{agent réducteur}} \text{créatine} + \text{APT}$$

agent réducteur : β-mercapto éthanol, glutathione réduite, cystéine, N-acétyl-cystéine ou dithiothréitol.
(2) pyruvate kinase :

$$\text{phosphoénol pyruvate} + \text{ADP} \xrightarrow{\text{Mn}^{++} \text{ ou } \text{Mg}^{++},\ \text{K}^{+}} \text{pyruvate} + \text{ATP}$$

(3) acétate kinase :

$$\text{acétylphosphate} + \text{ADP} \xrightarrow{\text{Mg}^{++} \text{ ou } \text{Mn}^{++}} \text{acétate} + \text{ATP}$$

(4) carbamate kinase :

$$\text{carbamylphosphate} + \text{ADP} \xrightarrow{\text{Mg}^{++}} NH_3 + CO_2 + \text{ATP}$$

(5) aspartate kinase :

$$\text{4-phospho-L-aspartate} + \text{ADP} \xrightarrow{\text{Mg}^{++}} \text{L-asparate} + \text{ATP}$$

(6) phosphoglycérate kinase :

1,3-diphospho-D-glycérate + ADP $\xrightarrow{Mg^{++} \text{ ou } Mn^{++}}$ 3-phospho-D-glycérate + ATP ; et

(7) arginine kinase :

arginine phosphate + ADP $\xrightarrow{Mg^{++} \text{ ou } Mn^{++}}$ L-arginine + ATP

**6.** Méthode selon l'une quelconque des revendications 1 à 4, où ladite ammoniaque ou ledit ion ammonium est le chlorure d'ammonium, de l'ammoniaque aqueuse, du sulfate d'ammonium, du nitrate d'ammonium, de l'acétate d'ammonium ou du citrate d'ammonium ou bien est généré ou consommé par une réaction enzymatique sélectionnée parmi :

(1) nicotine amidase :

$$\text{nicotine amide} + H_2O \rightarrow \text{nicotinate} + NH_3 + H^+$$

(2) glutaminyl-peptide glutaminase :

$$\text{L-glutaminyl-peptide} + H_2O \rightarrow \text{L-glutamyl-peptide} + NH_3$$

(3) arginine désaminase :

$$\text{L-arginine} + H_2O \rightarrow \text{citrulline} + NH_3 + H^+$$

(4) guanine désaminase :

$$\text{guanine} + H_2O \rightarrow \text{xanthine} + NH_3 + H^+$$

(5) adénosine désamidase :

$$\text{adénosine} + H_2O \rightarrow \text{inosine} + NH_3 + H^+$$

(6) créatinine désamidase :

$$\text{créatinine} + H_2O \rightarrow \text{N-méthylhydantoine} + NH_3 + H^+$$

(7) thréonine déshydratase :

$$\text{L-thréonine} + H_2O \rightarrow \text{2-oxobutyrate} + CO_2 + NH_3 + H^+$$

(8) aspartate ammonia-lyase :

$$\text{L-aspartate} \rightarrow \text{fumarate} + NH_3 + H^+$$

(9) L-méthionine-γ-lyase :

$$\text{L-méthionine} + H_2O \rightarrow \text{2-oxobutyrate} + CH_3SH + NH_3 + H^+;$$

et
(10) méthylamino-glutamate méthyl transférase :

$$\text{N-méthylglutamate} + NH_3 + H^{\cdot} \longrightarrow \text{glutamate} + \text{méthylamine}.$$

**7.** Méthode selon l'une quelconque des revendications 1 à 6, où ledit NAD produit est réduit en dit NAD réduit dans un système de réaction enzymatique comprenant de la déshydrogénase, qui consomme NAD et génère NAD réduit, et son substrat.

**8.** Méthode selon la revendication 7, où ladite déshydrogénase et son substrat sont sélectionnés parmi :

lactate déshydrogénase et L-lactate ;
alcool déshydrogénase et éthanol ;
glycérol-3-phosphate déshydrogénase et glycérol-3-phosphate ;
glucose déshydrogénase et glucose ;
malate déshydrogénase et L-malate ;
glutamate déshydrogénase et L-glutamate ; et
3-α-hydroxystéroide déshydrogénase et 3-α-hydroxystéroïde.

**9.** Méthode selon l'une quelconque des revendications 1 à 8, où ledit NAD réduit est oxydé en NAD dans un système réactionnel comprenant de l'oxydo-réductase, qui consomme NAD réduit et génère NAD oxydé et son substrat.

**10.** Méthode selon la revendication 9, où ladite oxydo-réductase et son substrat sont sélectionnés parmi :

lactate déshydrogénase et pyruvate ;
alcool déshydrogénase et acétaldéhyde ;
glycérol déshydrogénase et dihydroxyacétone ;
glycérol-3-phosphate déshydrogénase et
dihydroxyacétone phosphate ;
malate déshydrogénase et oxaloacétate ; et
3-α-hydroxystéroïde déshydrogénase et 3-cétostéroïde.

**11.** Méthode selon la revendication 9, où ladite oxydo-réductase et son substrat est NAD réduit : oxydo-réductase (accepteur) et son accepteur.

**12.** Méthode selon la revendication 11, où NAD réduit : oxydo-réductase (accepteur) et son accepteur sont NAD déshydrogénase réduite et un membre sélectionné parmi les flavines, les quinones, le 2,6-dichlorophénol indophénol, le sel de ferricyanure, le sel de tétrazolium, le cytochrome c et l'oxygène.

**13.** Méthode selon la revendication 9, où ladite oxydo-réductase et son substrat sont le sel de diaphorase tétrazolium et le résazurium.

**14.** Méthode selon l'une quelconque des revendications 1 à 8, où ledit NAD réduit est oxydé en NAD au moyen d'un agent de transfert d'électrons et de tétrazolium.

**15.** Méthode selon la revendication 14, où ledit agent de transfert d'électrons est le méthosulfate de phénazine, le bleu de meldola ou la pyrocyanine.

**16.** Méthode selon l'une quelconque des revendications 1 à 4, où on mesure AMP par un système de réaction enzymatique sélectionné parmi :

(1) adénylate kinase :

$$AMP + ATP \rightleftharpoons ADP + ADP$$

Pyruvate kinase :

$$ADP + \text{phosphoénol pyruvate} \rightleftharpoons ATP + \text{pyruvate}$$

pyruvate oxydase

$$\text{Pyruvate} + Pi + O_2 + H_2O \rightarrow \text{acétylphosphate} + CO_2 + H_2O_2$$

($H_2O_2$ produit est mesuré) ; et

(2) adénylate kinase :

$$AMP + ATP \rightleftharpoons ADP + ADP$$

Pyruvate kinase :

$$ADP + \text{phosphoénol pyruvate} \rightleftharpoons ATP + \text{pyruvate}$$

lactate déshydrogénase :

$$\text{Pyruvate} + NADH + H^+ \rightarrow \text{L-lactate} + NAD$$

(on mesure NADH diminué)

**17.** Méthode selon l'une quelconque des revendications 1 à 16, où ladite réaction de cyclage est effectuée en présence d'un agent tensioactif.

**18.** Méthode selon la revendication 17, où ledit agent tensioactif est un agent tensioactif non-ionique.